# EUROPEAN PATENT SPECIFICATION

(11) **EP 1 121 137 B1**
(45) Date of publication and mention of the grant of the patent: **20.07.2005**
(21) Application number: 99953196.5
(22) Date of filing: 15.10.1999
(51) Int. Cl.: A61K 35/76, C12N 7/00, A61P 35/00

(54) **METHOD FOR KILLING TUMOR AND TUMOR ASSOCIATED ENDOTHELIAL CELLS USING ADENOVIRAL MUTANTS**
VERFAHREN ZUM VERNICHTEN VON TUMOREN UND TUMOR-ASSOZIIERTEN ENDOTHELIALZELLEN MIT ADENOVIRALEN MUTANTEN
METHODE DE DESTRUCTION DE TUMEUR ET DE CELLULES ENDOTHELIALES ASSOCIEES A UNE TUMEUR A L'AIDE DE MUTANTS ADENOVIRAUX

(30) Priority: 26.10.1998 US 105701 P; 23.02.1999 US 121300 P
(43) Date of publication of application: 08.08.2001
(73) Proprietor: ONYX PHARMACEUTICALS, INC., Richmond, CA 94806 (US)
(72) Inventor: WILLIAMS, Angelica, Redwood City, CA 94065 (US); HEISE, Carla, Benecia, CA 94510 (US); PROPST, Meisa, Martinez, CA 94553 (US); SAMPSON-JOHANNES, Adam, Berkeley, CA 94708 (US); KIRN, David, Wimbledon, London, SW19 6DT (GB)
(74) Representative: Cripps, Joanna Elizabeth
(86) International application number: PCT/US1999/024144
(87) International publication number: WO 2000/024408

(56) References cited:
- WO-A-94/18992
- US-A- 5 801 029
- HEISE C ET AL: "ONYX-015, an E1B gene-attenuated adenovirus, causes tumor-specific cytolysis and antitumoral efficacy that can be augmented by standard chemotherapeutic agents." NATURE MEDICINE, vol. 3, no. 6, - June 1997 (1997-06) pages 639-45, XP002095383
- CHEN H ET AL: "Mapping of adenovirus 5 E1A domains responsible for suppression of neu-mediated transformation via transcriptional repression of neu." ONCOGENE, (1997 APR 24) 14 (16) 1965-71. , XP000891645
- WANG Z X ET AL: "Mutational analysis of the conserved region 2 site of adenovirus E1A and its effect on binding to the retinoblastoma gene product: use of the "double-tagging" assay." PROCEEDINGS OF THE NATIONAL ACADEMY OF SCIENCES OF THE UNITED STATES OF AMERICA, (1995 MAY 9) 92 (10) 4631-5. , XP002134552
- ABRAHAM S E ET AL: "Transforming growth factor beta 1 (TGF beta 1) reduces cellular levels of p34cdc2, and this effect is abrogated by adenovirus independently of the E1A-associated pRB binding activity." MOLECULAR BIOLOGY OF THE CELL, (1992 JUN) 3 (6) 655-65. , XP000891630
- HERMISTON T ET AL: "Generation of tumor-selective, replication competent adenoviruses." PROCEEDINGS OF THE AMERICAN ASSOCIATION FOR CANCER RESEARCH, vol. 40, March 1999 (1999-03), page 476 XP002134554
- RUMPF H ET AL: "Two domains within the adenovirus type 12 E1A unique spacer have disparate effects on the interaction of E1A with P105- Rb and the transformation of primary mouse cells." VIROLOGY, (1999 APR 25) 257 (1) 45-53. , XP002134555
- QUERIDO E ET AL: 'Accumulation of p53 induced by the Adenovirus E1A protein requires regions involved in the stimulation of DNA synthesis' JOURNAL OF VIROLOGY vol. 71, no. 5, May 1997, pages 3526 - 3533

## Description

### Field of the Invention

The invention described herein relates to the treatment or prevention of diseases involving hyperproliferative or uncontrolled cell growth, preferably cancer, using replication competent adenoviral mutants that selectively replicate in such cells compared to quiescent cells.

### Background of the Invention

The dominant features of human cancers are uncontrolled cell growth, and metastasis of the primary tumor. Recent work has shown that uncontrolled cell growth is a function of the loss of cell cycle checkpoint regulation. See, Hartwell, L.H. & Kastan, M.B., *Science* 266, 1821-1828 (1994). For instance, the retinoblastoma gene product (RB) and related proteins play key roles in preventing progression of normal cells from G1 into S phase until the appropriate proliferative signals are received. In contrast, RB function appears to be lost in almost all cancer cells, occurring as a result of RB or p16 gene mutation/deletion, cyclin D amplification/overexpression or cdk4 amplification/overexpression. See, Sherr. C.J. *Science* 274, 1672-1677 (1996). Consequently, a defect in the RB pathway fails to constrain cell growth, and the once normal cell assumes a malignant phenotype. Often associated with the malignant state is the capacity of a cancer cell to metastasize.

The adenovirus 5 E1A gene encodes two mRNAs having sedimentation values of 13S and 12S, which arise as a result of differential splicing of a common precursor, and encode proteins having 289 and 243 amino acids, respectively. The 46 amino acid difference between the two proteins is attributed to the 13S species. It is thought that E1A transforms cells by binding cellular RB, thus abrogating its tumor suppressor function.

The E1A gene has been shown to encode several different biological activities. In one instance, and in combination with either the middle T antigen of SV40 virus, or activated H-ras gene product, it is capable of transforming cells. In contrast, more recent work has shown that it can suppress the oncogenic phenotype associated with certain oncogenes. See, U.S. Patent No. 5,651,964. Mutations in the E1A gene have been identified that affect its anti-oncogenic activity, and certain of these have been shown to be effective in combination with chemotherapeutic drugs. See, WO 98/17806.

Frisch has shown an anti-oncogenic effect of adenovirus E1A in human tumor cells. See, Frisch, S. Proc. Natl. Acad. Sci. *USA* vol. 88, 9077-9081 (1991).

U. S. Patent No. 5,516,631 describes methods of inhibiting replication of hyperproliferative cells using nucleic acid encoding a polypeptide having adenovirus E1A activity.

WO 96/07322 describes a method of sensitizing tumor cells with adenoviral E1A to chemotherapeutic agents.

U.S. Patents Nos. 5,651,964; 5,643,567; 5,641,484; and WO 97/35012 describe methods for suppressing tumors that express the neu oncogene by the adenoviral E1A gene either alone or in combination with SV40 Large T antigen.

Others have shown that adenoviral E1A has an *in vivo* anti-tumor role. See, R. Sanchez-Prieto et al., Oncogene, vol. 13: 1083-92 (1996); and R. Sanchez-Prieto et al., Cancer Gene Therapy, vol. 5 (4) 215-224 (1998).

U.S. Patent No. 5,801,029 and WO 94/18992 describe methods for killing neoplastic cells by a replication competent adenovirus comprising an inactivating mutation in amino acids 120-139 of the E1A - CR2 domain.

Critical to tumor growth and metastasis is angiogenesis. Angiogenesis is necessary for growth and metastasis of the primary tumor as well as subsequent metastasis of secondary tumors. That is, a tumor cannot maintain sustained growth without a blood supply to provide nutrients, and remove metabolic wastes. Thus, formation of new blood vessels, or angiogenesis, facilitates tumor cell growth, allows tumor cells to gain entry to the blood stream, and to circulate throughout the body. Thus, inhibition or prevention of angiogenesis is expected to be an effective method for preventing tumor growth and metastasis.

It is worth noting that as part of the angiogenesis process, tumor-associated endothelial cells proliferate while endothelial cells associated with normal tissues are essentially quiescent. Therefore, therapeutic agents that target dividing cells, that is cancer cells, and their associated microvascular endothelial cells, would have enhanced antitumoral efficacy over agents that target either cell type alone. Clearly, a viral agent that has this property, that is one that replicates in the target cell thereby killing it, and in the process releases new viral particles capable of spreading and attacking additional target cells, would have major medical applications.

### Summary of the Invention

In accordance with the foregoing, the instant invention provides the use of a replication competent adenoviral mutants for the manufacture of a medicament for treating or preventing diseases of uncontrolled, hyperproliferative cell growth, and preferably those diseases that require or are facilitated by angiogenesis, including cancer.

In another aspect of the invention replication competent adenoviral mutants are capable of enhanced replication compared to wild type adenovirus in dividing normal or cancer cells, wherein the mutants exhibit a mutation in a RB family member binding region of E1A.

In yet another aspect of the invention replication competent adenoviral mutants are capable of enhanced cytopathogenicity compared to wild type adenovirus in dividing normal or cancer cells, wherein the mutants exhibit a mutation in a RB family member binding region of E1A.

In another aspect of the invention, replication competent adenoviral mutants that exhibit a mutation in the RB family member binding region of E1A, preferably the E1A-CR2 region, are capable of enhanced replication compared to wild type adenovirus in dividing normal or cancer cells.

In a further aspect of the invention, replication competent adenoviral mutants that exhibit a mutation in the RB family member binding region of E1A, preferably the E1A-CR2 region, are capable of enhanced cytopathogenicity compared to wild type adenovirus in dividing normal or cancer cells.

These and other objects of the present invention will become apparent to one of ordinary skill in the art upon reading the description of the various aspects of the invention in the following specification. The foregoing and other aspects of the present invention are explained in greater detail in the drawings, detailed description, and examples set forth below.

### Brief Description of the Figures

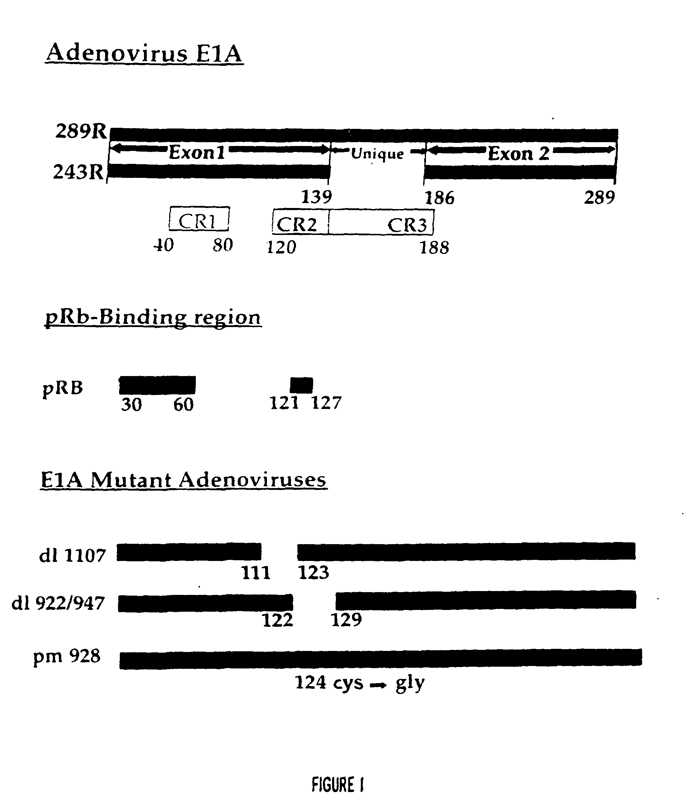

Fig. 1. Adenovirus E1A gene region. The conserved regions (CR) of E1A responsible for binding pRB (and p107, p130) are shown. The specific CR2 gene deletions present in d1922/947 and d11107, and point mutation in pm928 are shown.

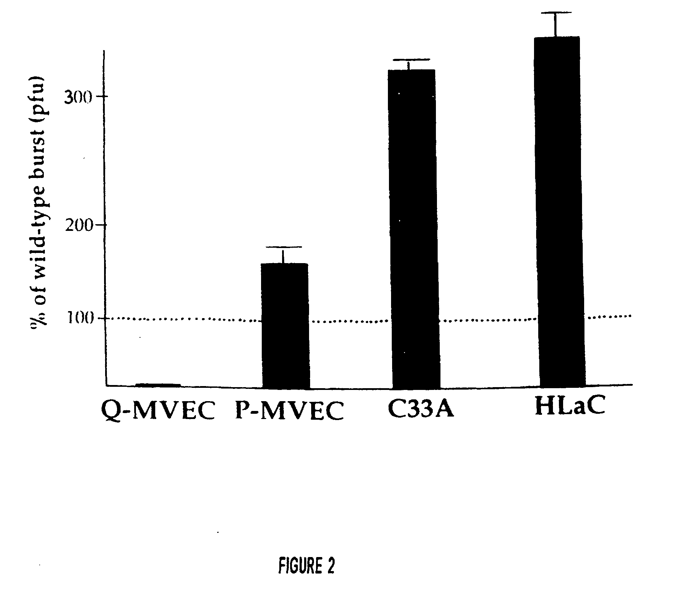

Fig. 2. Replication efficiency of an E1A-CR2 mutant (d1922/947) as a percentage of wild-type adenovirus replication. D1922/947 and wild-type adenovirus were tested for replication in quiescent or proliferating non-immortalized human microvascular endothelial cells (Q-MVEC and P-MVEC, respectively), C33A cervical carcinoma cells and HLaC laryngeal carcinoma cells. Cells were infected at an MOI of 10 pfu per cell and virus titers were determined 48 hours later by plaque assay on HEK 293 cells. Data are means (± SEM) of assays performed twice in triplicate.

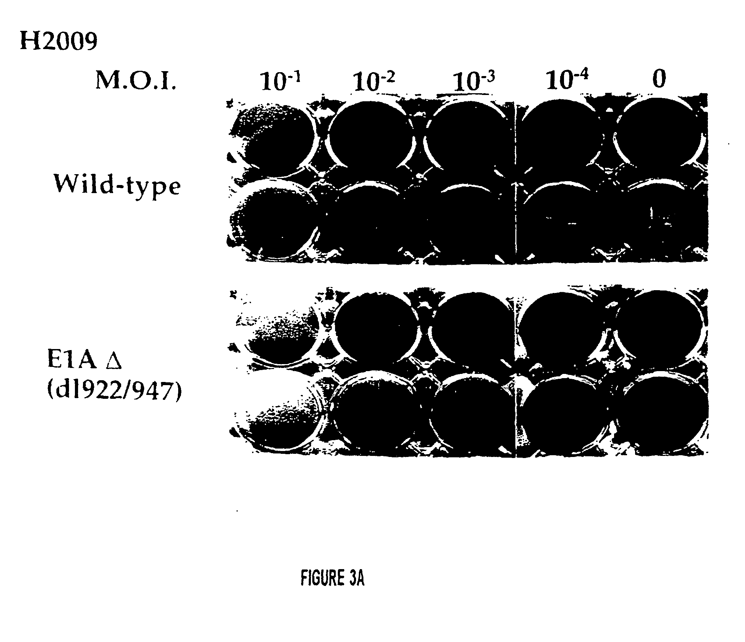

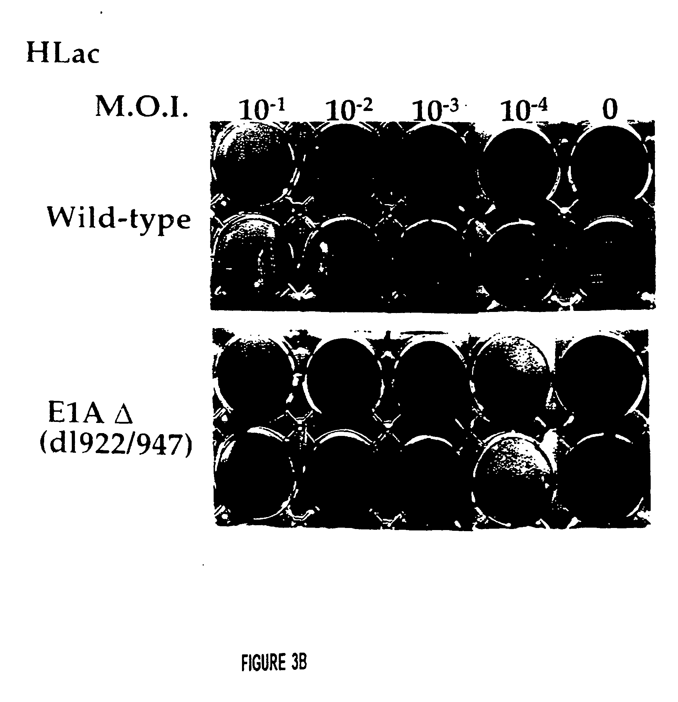

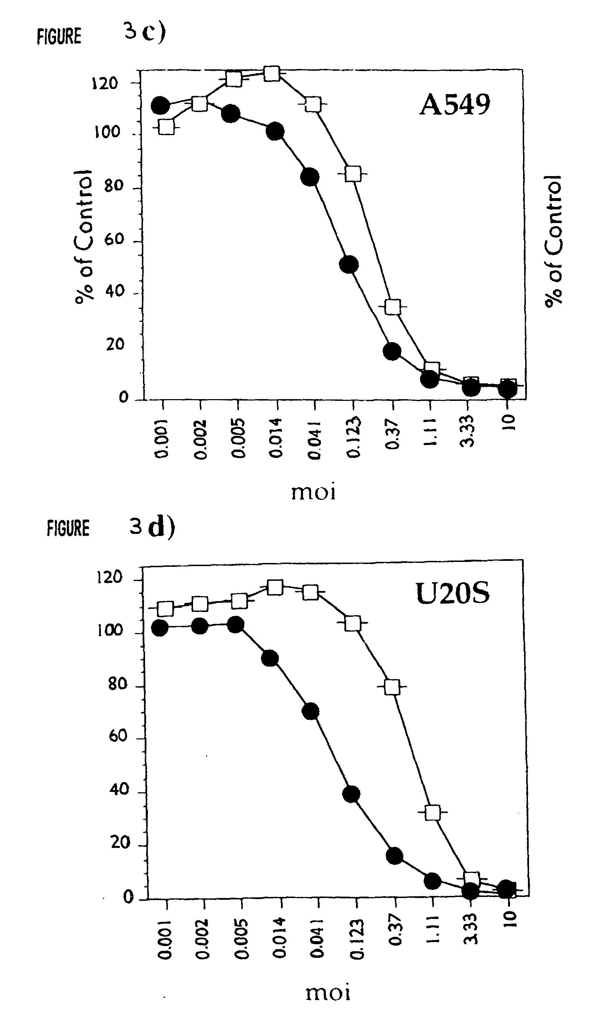

Fig. 3. Enhanced cytopathic effects of E1A-CR2 mutants (d1922/947 or d11107) versus wild-type adenovirus on tumor cells. Cells were infected at the multiplicites of infection (MOI) shown and 5 days later cell monolayers were fixed and stained. H2009 non-small cell lung carcinoma cells (panel A) and HLaC laryngeal carcinoma cells (panel B) were stained with crystal violet. A549 non-small cell carcinoma cells (panel C) and U20S osteosarcoma cells (panel D) were stained with sulforhodamine B; staining is expressed as a percentage of the uninfected control cell monolayers (open squares = wild-type adenovirus; closed circles = d11107). Assays were performed at least twice.

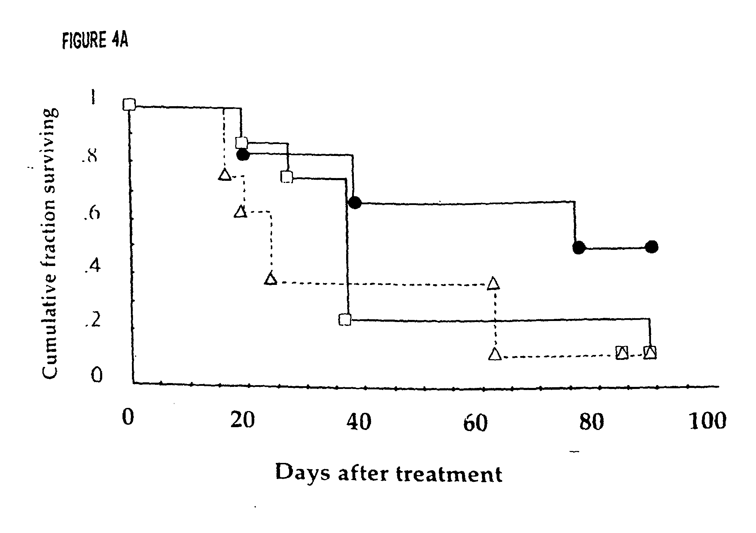

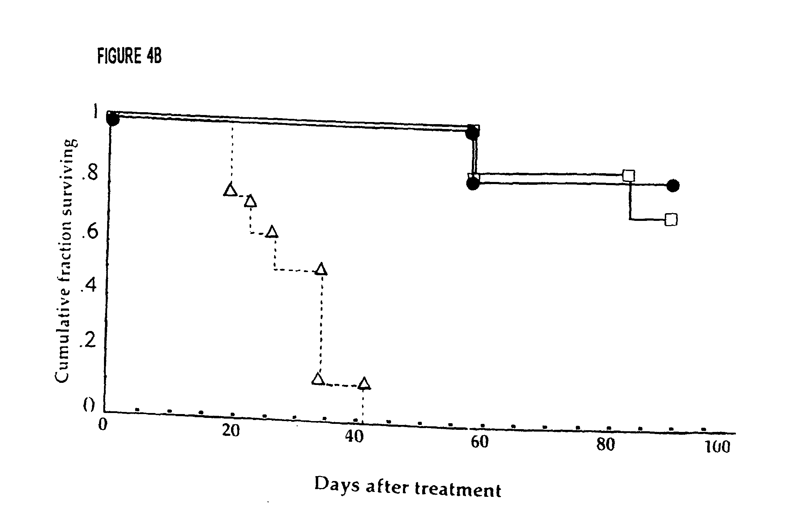

Fig. 4. Improved survival following intratumoral injection with E1A-CR2 mutant (d1922/947) in nude mouse-human tumor xenograft models. RKO.RC 7.14 human colon tumor cells (panel A) or HLaC human laryngeal tumor cells (panel B) were injected subcutaneously into the flanks of *nu*/*nu* mice and when tumors reached 5 to 7 mm maximal diameter they were injected with vehicle (open triangle) or 10⁸ pfu of either d1922/947 (closed circle) or wild-type adenovirus (open square) daily for five consecutive days (n = 6 - 13 per group). Tumors were measured twice weekly until sacrifice; animals were sacrificed after 90 days or once their tumor grew to a diameter of ≥ 1 cm. In the colon tumor xenograft model (panel A), the survival of the group injected with d1922/947 was significantly greater than the survival of either the vehicle- (p=0.0) or the wild-type adenovirus-injected (p=0.04) groups. In the laryngeal tumor xenograft model (panel B), the survival of both dl922/947- and wild-type adenovirus-treated groups were significantly greater than the vehicle-injected group (p≤0.001).

### Detailed Description of the Invention

### Definitions

Unless defined otherwise, all technical and scientific terms used herein have the same meaning as commonly understood by one of ordinary skill in the art to which this invention belongs. Generally, the nomenclature used herein and the laboratory procedures described below are those well known and commonly employed in the art. Standard techniques are used for recombinant nucleic acid methods, polynucleotide synthesis, and microbial culture and transformation (e.g., electroporation, lipofection). Generally enzymatic reactions and purification steps are performed according to the manufacturer's specifications. The techniques and procedures are generally performed according to conventional methods in the art and various general references which are provided throughout this document. See, Sambrook *et al*., Molecular Cloning: A Laboratory Manual, 2nd. edition (1989) Cold Spring Harbor Laboratory Press, Cold Spring Harbor, N.Y. The nomenclature used herein and the laboratory procedures in analytical chemistry, organic synthetic chemistry, and pharmaceutical formulation described below are those well known and commonly employed in the art. Standard techniques are used for chemical syntheses, chemical analyses, pharmaceutical formulation and delivery, and treatment of patients.

As employed throughout the disclosure, the following terms, unless otherwise indicated, shall be understood to have the following meanings:

The term "adenovirus" indicates over 40 adenoviral subtypes isolated from humans, and as many from other mammals and birds. See, Strauss, "Adenovirus infections in humans," in The Adenoviruses, Ginsberg, ed., Plenum Press, New York, NY, pp. 451-596 (1984). The term preferably applies to two human serotypes, Ad2 and Ad5.

"Neoplastic cells" or "neoplasia" refer to cells which exhibit relatively autonomous growth, so that they exhibit an aberrant growth phenotype characterized by a significant loss of control of cell proliferation. Neoplastic cells comprise cells which may be actively replicating or in a temporary non-replicative resting state (G₁ or G₀); similarly, neoplastic cells may comprise cells which have a well-differentiated phenotype, a poorly-differentiated phenotype, or a mixture of both type of cells. Thus, not all neoplastic cells are necessarily replicating cells at a given timepoint. The set defined as neoplastic cells consists of cells in benign neoplasms and cells in malignant (or frank) neoplasms. Herein, frankly neoplastic cells are frequently referred to as cancer, or cancer cells, tumor, or tumor cells, typically termed carcinoma if originating from cells of endodermal or ectodermal histological origin, or sarcoma if originating from cell types derived from mesoderm. Included within the definition of neoplastic cells are cells that lack p53 function, but are not frankly neoplastic. See, U.S. Patent No. 5,677,178. Examples of cells in the later category would be cells associated with Barret's syndrom, or leukoplakia.

"Replication competent adenovirus" or "replication competent mutant adenovirus" refers to an adenovirus that exhibits a mutation in a RB family member binding region of E1A, preferably the E1A-CR2 region. Dividing cells, to a greater degree than quiescent cells, infected with such a virus display one or more of the following phenotypic characteristics: (1) substantial expression of late gene products, such as capsid proteins (e.g., adenoviral penton base polypeptide) or RNA transcripts initiated from viral late gene promoter(s), (2) replication of viral genomes or formation of replicative intermediates, (3) assembly of viral capsids or packaged virion particles, or (4) appearance of cytopathic effect (CPE) in the infected cell, (5) completion of a viral lytic cycle.

"Physiological conditions," or "physiological solution" refers to an aqueous environment having an ionic strength, pH, and temperature substantially similar to conditions in an intact mammalian cell or in a tissue space or organ of a living mammal. Typically, physiological conditions comprise an aqueous solution having about 150 mM NaCl, pH 6.5-7.6, and a temperature of approximately 22-37° C. Generally, physiological conditions are suitable binding conditions for intermolecular association of biological macromolecules. For example, physiological conditions of 150 mM NaCl, pH 7.4, at 37°C are generally suitable.

By diseases involving "hyperproliferative" cell growth is meant pathologic conditions that are non-cancerous and characterized by unwanted cell growth such as endothelial cell growth associated with angiogenesis, or restenosis. Diseases involving "uncontrolled cell growth" typically refer to benign or metastatic cancer, respectively.

By "negative selection genes or agents" is intended a gene whose protein product enhances a host's ability to kill neoplastic cells. Such would include immunopotentiating agents, or enzymes that facilitate the conversion of a prodrug to its toxic metabolite.

Chemistry terms herein are used according to conventional usage in the art, as exemplified by The McGraw-Hill Dictionary of Chemical Terms (ed. Parker, S., 1985), McGraw-Hill, San Francisco.

DNA regions are operably linked when they are functionally related to each other. For example: a promoter is operably linked to a coding sequence if it controls the transcription of the sequence; a ribosome binding site is operably linked to a coding sequence if it is positioned so as to permit translation. Generally, operably linked means contiguous and, in the case of leader sequences, contiguous and in reading frame.

By replicating adenoviral vector is meant adenovirus or a mutant thereof that is capable of replicating in cancer cells. Such may include wild-type adenovirus, or, as discussed more in detail below, mutants of adenovirus that are capable of selectively replicating in certain types of cancer cells, preferably those that functionally lack one or more tumor suppressor proteins.

By "E1A RB family member binding regions", "RB binding site mutants" or "RB mutants" in adenovirus is meant those regions in the conserved region 2 (CR2) of E1A responsible for binding RB (p105), or two other E1A binding proteins, p107, and p130. Figure 1 shows the specific CR2 gene deletions present in the adenoviral mutants d1922/947, d11107, and the point mutation in pm928.

In another embodiment of the invention, an adenovirus that is mutant in an E1A RB family member binding region may also express a heterologous gene product, or negative selection agent, which can be toxic for the infected cells. A large selection of such agents may be used. For example, negative selection genes may be incorporated into a chosen adenovirus E1A RB family member binding region construct. A preferred embodiment is a HSV *tk* gene cassette operably linked to the E2 promoter of Ad5 NT d11110, or an alternative promoter and/or enhancer (e.g., major late promoter, E1a promoter/enhancer, E1b promoter/enhancer), with a polydenylation site to form a *tk* expression cassette. See, Zjilstra et al. (1989) Nature 342:435; Mansour et al. (1988) Nature 336: 348; Johnson et al. (1989) Science 245: 1234: Adair et al. (1989) Proc. Natl. Acad. Sci (U.S.A.) 86: 4574; Capecchi, M. (1989) Science 244:1288. The *tk* expression cassette (or other negative selection expression cassette) is inserted into the adenoviral genome, for example, as a replacement for a substantial deletion of the E3 gene. Other negative selection genes, including cytosine deaminase, will be apparent to those of skill in the art. See, U. S. Patent No. 5,358,866. It is believed that a negative selection gene operably linked to the E2 promoter is an especially preferred embodiment for incorporation into E1A RB binding mutants disclosed herein, and preferably the E1A-CR2 mutants.

In another embodiment, other negative selection agents would include cytokines, preferably an interleukin 1-15, more preferably IL-2 (U.S. Patent Nos. 4,738,927 or 5,641,665), interleukin 7, (U.S. Patent Nos. 4,965,195 or 5,328, 988), and IL-12 (See U.S. Patent No. 5,457,038), or an interferon, including interferon gamma (U.S. Patent Nos. 4,727,138 or 4,762,791). Also included would be tumor necrosis factor alpha, (U. S. Patent Nos. 4,677,063 or 5,773,582) or GM-CSF (U.S. Patent Nos. 5,393,870 or 5,391,485). Additionally, immunomodulatory proteins could be used and would include macrophage inflammatory proteins, including MIP-3, (See, Well, T. N. and Peitsch, MC. J. Leukoc. Biol vol 61 (5): pages 545-50,1997), and cell suicide, or apoptosis inducing proteins, including BAD and BAX. See, Yang, E., et al. Cell, vol 80, pages 285-291 (1995); and Sandeep, R., et al Cell, vol. 91, pages 231-241 (1997).

Although the effect of the instant compositions for treating cancer might be explained by one or more theories, it will be understood that the mechanism(s) by which the invention adenoviral mutants act, alone or in combination with chemotherapy, to treat hyperproliferative diseases, including cancer is presently not known. Thus, in discussing or alluding to such theories herein it will be understood that the inventors do not intend to be bound by them by having such considered limiting of the claims. Indeed, it is important to note that the invention E1A RB family member binding mutant adenoviruses, although being characterized by mutations in a RB family member binding region of E1A, exert their biological activity by mechanisms presently not known.

### Adenovirus

It is noteworthy that while the instant invention is described in terms of adenovirus type 5, it may be practiced with other similar adenovirus serotypes. The general organization of the adenoviral genome is conserved among serotypes, and specific functions are similarly situated. Further, the adenovirus 5 genome is registered as Genbank accession #M73260, and the virus is available from the American Type Culture Collection, Rockville, Maryland, U. S. A., under accession number VR-5. Methods for the construction of adenoviral mutants are generally known in the art. See, Mittal, S.K., Virus Res., 1993, vol: 28, pages 67-90. Certain of the materials and methods used to construct adenovirus mutants are described by Hanke, T., et. al. (1990) Virology, vol. 177, pages 437-444; and Bett, A.J., et. al., (993) J. Virol. vol. 67, pages 5911-5921, and in WO 96/40955. Microbix Biosystems, Inc., located at 341 Bering Avenue, Toronto, Ontario Canada, sells many of the materials used to construct adenovirus mutants, and provides Product Information Sheets on how to make them. Mutations in the E1A RB family member binding region are known in the art, and are readily generated using standard mutagenesis techniques, including PCR.

### Properties of E1A Mutant Adenovirus--Replication and Cytopathogenicity

DNA tumor viruses such as adenovirus are able to infect and replicate in quiescent cells because S phase entry is induced. This progression from G1 into S phase follows the inhibitory binding of RB and related cellular proteins by early viral gene products. See, Whyte, P., Williamson, N. & Harlow, E., *Cell* 56, 67-75 (1989); Moran, E., Zerler, B., Harison, T. & Mathews, M., *Molecular and Cellular Biology* 6, 3470-3480 (1986).

Binding and inactivation of RB (as well as p107, p 130, discussed more below) by adenovirus is facilitated by amino acids 121 to 127 of the E1A protein conserved region 2. See, Moran, E., Zerler, B., Harison, T. & Mathews, M. *Molecular and Cellular Biology* 6, 3470-3480 (1986); Whyte, P., Ruley, H. & Harlow, E., *J Virology* 62, 257-265 (1988). Mutations within this domain result in a loss of transformation by E1A without inhibiting its transactivation function. See, Moran, E., Zerler, B., Harison, T. & Mathews, M., *Molecular and Cellular Biology* 6, 3470-3480 (1986); Whyte, P., Ruley, H. & Harlow, E., *J Virology* 62, 257-265 (1988); Jelsma, T. *et al., Virology* 171, 120-130 (1989).

In one embodiment of the invention, adenovirus E1A RB binding site mutants are described that have reduced replication capacity in quiescent normal cells, but show enhanced replication in both cancer cells and proliferating normal cells, including, importantly, microvascular endothelial cells. Thus, in a mixed population of dividing and quiescent cells, viral replication substantially kills dividing cells while having much less effect on quiescent cells. In experiments described in the Examples, it is apparent that viruses with mutations in the RB family member binding region of E1A exhibit significantly reduced replication and cytopathogenicity in quiescent normal cells as compared to wild-type adenovirus. In contrast, and surprisingly, the replication and cytopathogenicity of the E1A RB family member binding site mutant viruses is greater than that seen with wild-type adenovirus in cancer cells and in proliferating normal cells, including microvascular endothelial cells. The following discussion presents a more detailed description of each of these properties, replication and cytopathogenicity, of the invention adenoviral mutants in various cell types, either dividing or quiescent.

The instant invention presented in detail below, and particularly in the Examples, centers on the results of experiments with three adenoviral mutants with well characterized mutations in the E1A RB family member binding region, conserved region 2 (CR2) of the virus. The viruses are denoted: d11107, d1922/947 and pm928.

The CR2 region of wild type adenovirus binds three cellular proteins; p130, p107, and p105. p105 corresponds to RB. Previous work by others {(See, Barbeau, D., et al. Oncogene, vol. 9, 359-373 (1994)}, and our unpublished observations, have elucidated the effect of mutations in the E1A CR2 region of the adenoviral mutants, d11107, d1922/947 and pm928 on binding to these cellular proteins. E1A encoded by d11007 binds substantially only to p107, whereas E1A encoded by d1922/947 and pm928 show little or no binding to any of the three cellular proteins. It thus appears that the results presented below apply to adenoviral mutants having at least one mutation in what is referred to herein as E1A RB binding site region.

### Replication of E1A-CR2 Mutant Adenoviruses in vitro Relative to Wild-Type

In one embodiment of the invention, adenoviral mutants with well characterized mutations in E1A RB family member binding region, and preferably in the E1A RB binding conserved region 2 (CR2) of the virus, are shown to replicate in a panel of normal cells and cancer cells (Figure 2). More preferred, one such adenovirus E1A-CR2 RB binding site mutant, d1922/947, was unable to replicate to a titer above the input titer in quiescent, non-immortalized human microvascular endothelial cells (MVEC). In contrast, and as discussed more in the Examples, such E1A RB binding site mutant viruses replicate significantly better than wild-type adenovirus in actively proliferating MVECs.

Further, the replication of these E1A RB family member binding mutant adenoviruses in human tumor cells with mutant RB (C33A, cervical) or normal RB expression (HLaC, laryngeal) was determined. The E1A RB binding site mutant viruses replicated better than wild-type adenovirus in both tumor cell lines. Similar results were obtained in A549 carcinoma cells that have normal RB expression.

### Cytopathic effect in RB+ cell lines

In a second embodiment of the invention, adenoviral mutants with well characterized mutations in the E1A RB family member binding region, and preferably in the E1A RB binding site conserved region 2 (CR2) of the virus, are shown to exhibit enhanced cytopathogenicity versus wild-type adenovirus on all tumor cell lines tested, including cells with normal RB expression (HLaC, RKO, H1299, U20S and A549), and those with abnormal RB expression (C33A, H2009) (Figure 3). The viral dose required to destroy 50 percent of a cell monolayer was up to ten-fold higher for wild-type adenovirus than for the E1A-CR2 RB binding site mutants, d11107 or d1922/947. Similarly, proliferating MVECs, small airway epithelial cells (SAECs), and mammary epithelial cells were shown to be more sensitive to CPE with the E1A-CR2 mutants than to wild-type virus. The presence of wild-type RB protein appeared to influence CPE results. For instance, the superior cytopathogenicity of E1A-CR2 RB family member binding mutants relative to wild-type adenovirus was greatest in cells with normal RB expression. In contrast, quiescent MVECs and mammary epithelial cells (MECs) were less sensitive to the E1A RB binding mutant viruses than to wild-type adenovirus.

### E1A mutants show enhanced efficacy against tumors in vivo.

In yet another embodiment of the invention the E1A RB family member binding site mutant viruses, and preferably the E1A-CR2 RB mutants, were shown to have superior or equivalent antitumoral activity in vivo compared to that seen with wild-type adenovirus (Figure 4).

The antitumoral efficacy was determined using human tumor xenografts in athymic mice as described in the Examples. For example, animals bearing colon tumors that were injected with an E1A-CR2 RB family member binding site mutant virus had a median survival of 80 days versus 35 days in the group treated with wild-type adenovirus, and 25 days for the vehicle-control group. Additional results are presented in the Examples.

### E1A mutants show decreased replication and toxicity in quiescent normal cells in vivo compared to wild type virus.

The cotton rat is a permissive host for human adenovirus (See, Ginsberg, H.S. *et al., Proc Natl Acad Sci U S A* 86, 3823-3827 (1989) and its lung is an established model to study adenoviral replication and pathology *in vivo. See,* Prince, G.A. *et al., J Virol* 67, 101-111 (1993); Ginsberg, H.S. & Prince, G.A., *Infect Agents Dis* 3, 1-8 (1994). Thus, experiments were conducted in Cotton rats that confirmed the substantial resistance of quiescent normal cells to the E1A RB family member binding site mutant viruses versus wild-type adenovirus. *In situ* hybridization of the lungs infected with E1A RB binding site mutant virus demonstrated no replication in one case and low level replication in the other cases. In contrast, *in situ* hybridization of the lungs infected with wild-type adenovirus demonstrated higher level replication in all animals. Wild-type adenovirus infected cells diffusely throughout the bronchiolar epithelium and within the lung parenchyma, whereas E1A RB family member mutant virus replication was limited to isolated bronchiolar epithelial cells. These results are presented in detail in the Examples.

Thus, taken together the preferred embodiment of the invention is adenoviral E1A RB family member binding mutants, and more preferred are adenoviral E1A-CR2 RB family member mutants, that are mutated in the RB family member binding region of E1A. These viruses show reduced replication in quiescent normal cells, while replication and cytopathogenicity is greater than wild-type adenovirus in cancer cells and proliferating endothelial cells.

The enhanced replication and cytopathogenicity of E1A-CR2 RB binding site mutants versus wild-type adenovirus in proliferating cells was unexpected. Indeed, several adenovirus and herpes virus mutants are known that have been genetically attenuated in order to achieve selective replication in tumor cells. See, Heise, C. *et al., Nat. Med.* 3, 639-645 (1997); Bischoff, J.R. *et al., Science* 274, 373-376 (1996); Martuza, R.L., *et al., Science* 252, 854-856 (1991); Mineta, T., *Nat Med* 1, 938-943 (1995). Each of these attenuated viruses replicates less efficiently than its wild-type parental virus, even in tumor cells. See, Bischoff, J.R. *et al., Science* 274, 373-376 (1996); Martuza, R.L. *et al., Science* 252, 854-856 (1991); Kirn, D.H., *Expert Opinion on Investigational Drugs* 5, 753-762 (1996). In some cases replication can be reduced by 10 to 100-fold versus the wild-type virus. See, Martuza, R.L. et al., *Science* 252, 854-856 (1991). This is presumably due to the loss of important viral functions that enhance replication. The reason for the enhanced replication of E1A-CR2 RB binding mutants versus wild-type adenovirus is unknown.

It will be apparent to the skilled practitioner of this art that the E1A RB binding site mutants disclosed herein, and preferably the E1A-CR2 RB binding site mutants, have a large therapeutic index between dividing and quiescent cells, or more specifically, tumor and proliferating microvascular endothelial cells, and quiescent microvascular endothelial cells.

It will thus be further appreciated that this preferred embodiment of the invention can be beneficially applied to substantially, and selectively kill dividing tumor cells directly by adenoviral replication and cytopathogenic effect therein. This effect is enhanced by the elimination of developing blood vessels by the adenoviral E1A Rb binding site mutants. Both antitumor effects occur with minimal killing of normal quiescent cells.

### Formulations/Administration

The adenoviral mutants described herein, may be formulated for therapeutic and diagnostic administration to a patient. For therapeutic or prophylactic uses, a sterile composition containing a pharmacologically effective dosage of adenovirus is administered to a human patient or veterinary non-human patient for treatment, for example, of a neoplastic condition. Generally, the composition will comprise about 10³ to 10¹⁵ or more adenovirus particles in an aqueous suspension. A pharmaceutically acceptable carrier or excipient is often employed in such sterile compositions. A variety of aqueous solutions can be used, e.g., water, buffered water, 0.4% saline, 0.3% glycine and the like. These solutions are sterile and generally free of particulate matter other than the desired adenoviral vector. The compositions may contain pharmaceutically acceptable auxiliary substances as required to approximate physiological conditions such as pH adjusting and buffering agents, toxicity adjusting agents and the like, for example sodium acetate, sodium chloride, potassium chloride, calcium chloride, sodium lactate, etc. Excipients which enhance infection of cells by adenovirus may be included, preferably polycations.

Adenoviruses of the invention, or the DNA contained therein, may also be delivered to neoplastic cells by liposome or immunoliposome delivery; such delivery may be selectively targeted to neoplastic cells on the basis of a cell surface property present on the neoplastic cell population (e.g., the presence of a cell surface protein which binds an immunoglobulin in an immunoliposome). Typically, an aqueous suspension containing the virions are encapsulated in liposomes or immunoliposomes. For example, a suspension of adenovirus virions can be encapsulated in micelles to form immunoliposomes by conventional methods (U.S. Patent 5,043,164, U.S. Patent 4,957,735, U.S. Patent 4,925,661; Connor and Huang (1985) J. Cell Biol. 101: 582; Lasic DD (1992) Nature 355: 279; Novel Drug Delivery (eds. Prescott LF and Nimmo WS: Wiley, New York, 1989); Reddy et al. (1992) J. Immunol. 148: page 1585). Immunoliposomes comprising an antibody that binds specifically to a cancer cell antigen (e.g., CALLA, CEA) present on the cancer cells of the individual may be used to target virions, or virion DNA to those cells.

The compositions containing the present adenoviruses or cocktails thereof can be administered for prophylactic and/or therapeutic treatments of neoplastic disease. In therapeutic application, compositions are administered to a patient already affected by the particular neoplastic disease, in an amount sufficient to cure or at least partially arrest the condition and its complications. An amount adequate to accomplish this is defined as a "therapeutically effective dose" or "efficacious dose." Amounts effective for this use will depend upon the severity of the condition, the general state of the patient, and the route of administration. Single or multiple administrations of the compositions can be carried out with dose levels and pattern being selected by the treating physician. In any event, the pharmaceutical formulations should provide a quantity of E1A RB family member binding mutant adenoviruses of this invention sufficient to effectively treat the patient.

In prophylactic applications, compositions containing the invention adenoviruses, or cocktails thereof, are administered to a patient not presently in a neoplastic disease state to enhance the patient's resistance to recurrence of a cancer or to prolong remission time. Such an amount is defined to be a "prophylactically effective dose." In this use, the precise amounts again depend upon the patient's state of health and general level of immunity.

Adenoviral therapy of the present invention may be combined with other antineoplastic protocols, such as conventional chemotherapy, or with other viruses. See U.S. Patent No. 5,677,178, issued October 14, 1997. Chemotherapy may be administered by methods well known to the skilled practitioner, including systemically, direct injection into the cancer, or by localization at the site of the cancer by associating the desired chemotherapeutic agent with an appropriate slow release material or intra-arterial perfusing the tumor. The preferred chemotherapeutic agent is cisplatin, and the preferred dose may be chosen by the practitioner based on the nature of the cancer to be treated, and other factors routinely considered in administering cisplatin. Preferably, cisplatin will be administered intravenously at a dose of 50-120 mg/m² over 3-6 hours. More preferably it is administered intravenously at a dose of 80 mg/m² over 4 hours. A second chemotherapeutic agent, which is preferably administered in combination with cisplatin is 5-fluorouracil. The preferred dose of 5-fluorouracil is 800-1200 mg/m² per day for 5 consecutive days.

Adenoviral therapy using the instant invention adenoviruses may be combined with other antineoplastic protocols, such as gene therapy. See, U.S. Patent No. 5, 648, 478. As mentioned above, adenovirus constructs for use in the instant invention will exhibit specific cancer cell killing, preferably though the expression of pro-drug activator genes driven off a tissue specific promoter. See, U.S. Patent No. 5, 631, 236.

Also, in the event that the instant invention adenoviral mutants elicit an immune response that dampens their effect in a host animal, they can be administered with an appropriate immunosuppressive drug to maximize their effect. Alternately, a variety of methods exist whereby the exterior protein coat of adenovirus can be modified to produce less immunogenic virus. See, WO 98/40509. There it is shown that a major immunogenic component of adenovirus' exterior coat, hexon protein, can be genetically engineered to be less immunogenic. This is done by creating a chimeric hexon protein by substituting for normal viral hexon protein epitopes a sequence of amino acids not normally found in hexon protein. Such adenoviral constructs are less immunogenic than the wild type virus.

To increase the efficacy of the invention adenoviral E1A mutant constructs they may be modified to exhibit enhanced tropism for particular tumor cell types. For example, as shown in WO 98/40508 a protein on the exterior coat of adenovirus may be modified to display a chemical agent, preferably a polypeptide, that binds to a receptor present on tumor cells to a greater degree than normal cells. Also see, U.S. Patent No. 5,770,442 and U.S. Patent No. 5,712,136. The polypeptide can be antibody, and preferably is single chain antibody.

### Purification of Adenoviral Mutants

Adenovirus is routinely purified by a number of techniques including cesium chloride banding using an ultracentrifuge. However, for large scale production of adenovirus, methods which give larger yields than those readily obtainable by cesium chloride ultracentrifugation are desirable, and involve one or more chromatographic steps. The preferred method utilizes ion exchange chromatography. See, for example, WO 98/22588; and Huyghe et al., Human Gene Therapy, vol. 6: 1403-1416 (1996).

The Examples which follow are believed to be exemplary of the invention, but it will be appreciated by those skilled in the art that many modifications and alterations may be made to these embodiments without departing from the scope of the invention.

### Example 1

### Cytopathogenicity of Adenoviral E1A-CR2 RB Family Member Binding Mutants

The cytopathogenicity of adenoviral E1A-CR2 RB family member binding mutants was tested using three mutants. D1922/947 has a deletion of amino acids 122 to 129 {See, Whyte, P., Ruley, H. & Harlow, E., *J Virology* 62, 257-265 (1988)}, whereas d11107 has a deletion of amino acids 111 to 123 {See, Jelsma, T. *et al., Virology* 171, 120-130 (1989)}. Pm928 has a point mutation resulting in conversion of amino acid 124 from a cysteine to a glycine. See, Moran, E., *et al., Molecular and Cellular Biology* 6, 3470-3480 (1986). Both d1922/947 and pm928 have been shown to be defective in transformation despite normal E1A expression levels. Ad5 and wild-type D were used as wild-type adenovirus controls; these viruses are nearly identical except for deletions in the E3 region in wild-type D. See, Barker, D.D., *et al., Virology* 156, 107-121 (1987).

For assays using proliferating cells, cells were grown to 70% confluence (2% FBS) at which time cells were infected with multiplicities of infection (MOI) of 0.001 to 10. Assays were followed for five days. At this time plates were either a) stained with crystal violet and photographed, or b) stained with sulforhodamine B (SRB) and tested by colorimetric assay. The SRB assay has been described previously. See, Skehan, P., *et al., Proc Am Assoc Cancer Res* 30, 2436 (1989). Briefly, cells were plated in 96-well plates in quadruplicate and allowed to adhere for 6 hours. Serially diluted virus was added to test wells and the plates were incubated at 37°C in a humidified incubator with 5% CO₂. The assay was stopped on the appropriate day post-infection by the addition of trichloroacetic acid to a final concentration of 10% for a minimum of one hour at 4°C. Plates were then washed with deionized water and allowed to air dry. Cells remaining on the plates were stained with a 0.2% SRB solution in 1% acetic acid for 20 minutes at room temperature, washed with 1% acetic acid, and air dried. Bound dye was solubilized with 10mM unbuffered Tris base for 5-10 minutes on a shaker. Absorbance was read at 515 nm on a plate reader. Percent of control values was calculated as the optical density (O.D.) of test wells/ O.D. of mock controls and plotted versus viral multiplicity of infection (MOI). Normal cells were made quiescent following growth to complete confluence and subsequent growth in 0.2 % FBS. Quiescence was confirmed by cell cycle analysis and cell counts before each assay.

The following tumor cell types were used in the cytopathogenicity assays, and were obtained from the ATCC: non-small cell lung carcinoma (A549, H2009, H1299), colon carcinoma (RKO), cervical carcinoma (C33A) and laryngeal carcinoma (HLaC). Cells were grown as previously described. See, Bischoff, J.R. *et al*., Science 274, 373-376 (1996). RKO human colon tumor cells expressing the human papillomavirus E7 gene (RKO-RC 7.14) were obtained from Dr. Kathy Cho, John's Hopkins University.

The results showed that both d1922/947 and d11107 caused enhanced cytopathogenicity versus wild-type adenovirus on all tumor cell lines tested, including cells with normal RB expression (HLaC, RKO, H1299, U2OS and A549) and those with abnormal RB expression (C33A, H2009) (Figure 3). Using quantitative sulforhodamine B assays (SRB), the viral dose required to destroy 50 percent of the monolayer was up to ten-fold higher for wild-type adenovirus than for d11107. Similarly, proliferating MVECs, small airway epithelial cells (SAECs), and MECs (obtainable from Clonetics, San Diego, CA) were more sensitive to CPE with the E1A-CR2 RB binding site mutants than to wild-type virus. The rate of CPE induction with the point mutant pm928 was intermediate between that seen with the two E1A-CR2 RB binding site deletion mutants and wild-type adenovirus. Interestingly, the presence of wild-type RB protein appeared to influence CPE results; the superior cytopathogenicity of the E1A-CR2 RB binding site mutants relative to wild-type adenovirus was greatest in cells with normal RB expression. In contrast, quiescent MVECs and MECs were less sensitive to the E1A mutant viruses than to wild-type adenovirus.

Thus, these data clearly support the utility of the adenoviral E1A-CR2 RB family member binding site mutants to kill tumor cells directly, or by eliminating their underlying blood vessel nutrient endothelial cell support structure.

### Example 2

### Replication of Adenoviral E1A-CR2 RB Family Member Binding Site Mutants

Viral replication assays were carried out as previously described. See, Bischoff, J.R. *et al*., Science 274, 373-376 (1996). Briefly, cells were grown as described for the cytopathic effect assays in Example 1, and were infected at a MOI of 10 with each virus. Forty-eight hours later, both cells and supernatant were harvested for virus titration analysis. Cell lysates underwent three cycles of freezing and thawing, followed by a 30-s pulse in a sonicator water bath. Serial dilutions of supernatants and lysates were titered on HEK293 cells (human embryonic kidney cells expressing the E1 region of Ad2).

Dl922/947 was unable to replicate to a titer above the input titer in quiescent, non-immortalized human microvascular endothelial cells (MVEC) (Figure 2). In contrast, both E1A mutant viruses, d11107 and pm928, replicated significantly better than wild-type adenovirus in actively proliferating MVECs. Similar results were obtained with quiescent and proliferating human mammary epithelial cells (data not shown). The replication of dl922/947 was increased in proliferating versus non-proliferating endothelial cells by approximately 25-fold (7 x 10⁷ versus 2.6 x 10⁶ pfu/ml) versus a 2-fold difference for wild-type adenovirus (4 x 10⁷ versus 2 x 10⁷ pfu/ml).

We next evaluated the replication of these viruses in human tumor cells with mutant RB (C33A, cervical) or normal RB expression (HLaC, laryngeal). The three E1A RB binding mutant viruses replicated better than wild-type adenovirus in both tumor cell lines. Similar results were obtained in A549 carcinoma cells (normal RB expression).

### Example 3

### Adenoviral E1A-CR2 RB Family Member Binding Site Mutants Show Enhanced Efficacy in Nude Mouse-Human Tumor Xenograft Models

We evaluated the antitumoral efficacy of dl922/947, pm928 and wild-type adenovirus against human tumor xenografts in athymic mice. Female athymic mice (Hsd: Athymic Nude-nu) were obtained at 4 to 6 weeks of age from the Harlan Sprague-Dawley Company and were quarantined for at least two weeks prior to the study. RKO-RC 7.14 or HLaC human carcinoma cells (2 x 10⁶ cells) were injected subcutaneously in the flanks of 6 to 8 week old mice. When tumors grew to a maximal diameter of 5 to 7 mm, direct intratumoral administration was carried out as follows.

Tumors were injected directly with 10⁸ pfu of virus suspended in 60 µl PBS each day for five consecutive days (n=6-13 per group). Vehicle-treated control tumors received PBS alone in identical fashion. Tumor sizes were determined twice weekly until animals were sacrificed (tumor volume > 1 cm³ or 90 days after treatment). Tumor cells were injected with either an E1A-CR2 RB binding site mutant (pm928 or dl922/947), wild-type adenovirus or vehicle. Animals bearing colon tumors that were injected with dl922/947 had a median survival of 80 days versus 35 days in the group treated with wild-type adenovirus and 25 days for the vehicle-control group (p= 0.04 for dl922/947 versus wild-type) (Figure 4, panel A). The median survival of HLaC tumor-bearing animals treated with either dl922/947 or wild-type adenovirus was not reached through 90 days following treatment (80% and 70% remained alive, respectively); the median survival of the vehicle-control group was 30 days (Figure 4, panel B). Complete tumor regressions occurred in 8 of 12 HLaC tumors treated with d1922/947, 5 of 13 treated with pm928, 3 of 7 treated with wild-type adenovirus and in 0 of 8 injected with vehicle. None of the mice with complete tumor regressions had evidence of tumor recurrence at the time of study termination (90 days). Tumors harvested up to 70 days after treatment with the E1A CR2 RB family member binding site mutants contained cells with actively replicating virus detected by in situ hybridization for adenoviral DNA. Similar survival and response data were also seen in C33A tumor xenografts (data not shown).

In situ hybridization was performed on formalin-fixed, paraffin embedded tissue, cut into 5 µm sections. Slides were deparaffinized in xylenes, hydrated through ethanols, digested with proteinase K and post-fixed in 4% paraformaldehyde. Hybridization was carried out overnight at 37°C with 0.5 µg/ml biotinylated adenovirus DNA probe (Enzo Diagnostics, Inc. Farmingdale, NY). After 3 successive washes in 1X SSC at 55°C, an alkaline phosphatase conjugated-anti-biotin antibody (Vector Laboratories) was applied. NBT/BCIP was used as the chromagen and slides were counterstained with nuclear fast red.

### EXAMPLE 4

### E1A-CR2 RB Family Member Mutants Show Decreased Replication and Toxcity in Quiescent Cotton Rat Lung Cells Compared to Wild Type Virus

The cotton rat is a permissive host for human adenovirus. See, Ginsberg, H.S. et al., Proc Natl Acad Sci U S A 86, 3823-3827 (1989). Its lung is an established model to study adenoviral replication and pathology *in vivo.* See, Prince, *G.A. et al*., J Virol 67, 101-111 (1993); Ginsberg, H.S. & Prince, G.A., Infect Agents Dis 3, 1-8 (1994). Cotton rats were given intranasal inoculations of either d1922/947 or wild-type adenovirus and were sacrificed three or 14 days later (n= 5 per group/ timepoint). The relative resistance of quiescent normal cells to d1922/947 versus wild-type adenovirus was confirmed in this model.

In situ hybridization (performed as described in Example 3) of the lungs infected with d1922/947 demonstrated no replication in one case and low level replication in the other cases on day 3 post-infection. In contrast, in situ hybridization of the lungs infected with wild-type adenovirus demonstrated higher level replication in all animals. Wild-type adenovirus infected cells diffusely throughout the bronchiolar epithelium and within the lung parenchyma, whereas d1922/947 replication was limited to isolated bronchiolar epithelial cells. The alveolar histopathology was also reduced in the d1922/947 infected lungs versus wild-type infected lungs both 3 days and 14 days after infection.

Cotton rats (Sigmodon hispidus) were infected with adenoviruses by intranasal inoculation as previously described. See, Ginsberg, H.S. et al., Proc Natl Acad Sci U S A 86, 3823-3827 (1989); Prince, G.A., et al., J Virol 67, 101-111 (1993); Ginsberg, H.S. & Prince, G.A., Infect Agents Dis 3, 1-8 (1994). Briefly, animals were inoculated with 10⁹ pfu intranasally (n=5 per treatment group) and sacrificed three days later; previous experiments have shown that this is the peak time for adenoviral replication in this animal model. Lungs were processed and sectioned as described above for in situ hybridization.

### EXAMPLE 6

### S-Phase Induction and Viral DNA Synthesis of RB Binding Site Mutants is Reduced in Quiescent Normal Cells Versus Wild-Type Adenovirus

The ability of the Rb binding site mutant adenoviruses, d1922/947 and d11107, to induce S-phase in non-proliferating normal cells was compared to wild-type adenovirus. At baseline only 10% of the non-immortalized small airway epithelial cells were in S-phase. Twenty-four hours after infection with wild-type adenovirus (m.o.i. of 10), 61% of cells were in S-phase; d11107 infection led to S-phase in 40% of these cells. However, d1922/947 was significantly less effective at inducing S-phase; only 26% of cells were in S-phase after twenty-four hours. Viral DNA replication was estimated by dot blot analysis in the same assay after twenty-four hours. Although viral DNA production was roughly equivalent for wild-type adenovirus and d11107, the viral DNA replication documented with d1922/947 was significantly less (approximately 30 % of wild-type levels; p< 0.05).

From the foregoing description, one skilled in the art can easily ascertain the essential characteristics of the instant invention, and without departing from the scope thereof, can make various changes and modifications of the invention to adapt it to various usages and conditions.

## Claims

1. An in vitro method for substantially and selectively killing dividing cells, in a cell population comprising dividing and quiescent cells, said method comprising: contacting said cell population under infective conditions with a replication competent adenovirus comprising a mutation in the E1A RB family member binding region of said adenovirus, and allowing sufficient time for said adenovirus to infect said cell population, wherein said mutation is in the E1A-CR2 region and comprises a deletion or substitution of one or more of (a) amino acids 122 through 129; or (b) amino acids 111 through 123.

2. A method as described in claim 1 wherein said dividing cells are cancer cells.

3. A method as described in claim 2 wherein said dividing cells are endothelial cells.

4. A method as described in claim 3 wherein said quiescent cells are endothelial cells.

5. A method as described in any one of claims 1 to 4 wherein said adenovirus is d1922/947.

6. A method as described in any one of claims 1 to 4 wherein said adenovirus is d11107.

7. A method as described in any one of claims 1 to 4 wherein said adenovirus is pm928.

8. An in vitro method for killing dividing endothelial cells with substantially less killing of quiescent endothelial cells, in a cell population comprising dividing and quiescent endothelial cells, said method comprising contacting said cell population under infective conditions with a replication competent adenovirus, said adenovirus comprising a mutation in an E1A CR2 RB family member binding region of said adenovirus, and allowing sufficient time for said mutant adenovirus to infect said cell population, wherein said mutant adenovirus replicates to higher titers in said dividing cells than wild type adenovirus.

9. An in vitro method for substantially and selectively killing dividing endothelial cells and cancer cells compared to quiescent endothelial cells in a cell population comprising said three cell types, said method comprising contacting said cell population under infective conditions with a replication competent adenovirus comprising a mutation in an E1A CR2 RB family member binding region of said adenovirus, and allowing sufficient time for said mutant adenovirus to infect said cell population.

10. A method as described in claim 9 wherein said dividing endothelial cells are microvascular endothelial cells.

11. A method as described in claim 10 wherein said adenovirus mutation comprises a mutation in the E1A-CR2 region.

12. Use of a replication competent adenovirus comprising a mutation the E1A CR2 region of said adenovirus, said mutation comprising a deletion of substitution of one or more of (a) amino acids 122 through 129; or (b) amino acids 111 through 123; in the manufacture of a medicament for use in the treatment or prevention of a disease of uncontrolled or hyperproliferative cell growth.

13. Use according to claim 12 wherein said adenovirus is d1922/947.

14. Use according to claim 12 wherein said adenovirus is d11107.

15. Use according to claim 12 wherein said adenovirus is pm928.

16. Use of a replication competent adenovirus comprising a mutation in the E1A RB family member binding region, in the manufacture of a medicament for controlling angiogenesis in an animal, by killing dividing microvascular endothelial cells.

17. Use according to claim 16 wherein said E1A RB family member binding region of said adenovirus is in the E1A-CR2 region.

18. Use according to claim 17 wherein said mutation in the E1A-CR2 region comprises a deletion or substitution of one or more amino acids 122 through 129.

19. Use according to claim 18 wherein said mutation in the E1A-CR2 region comprises a deletion or substitution of one or more amino acids 111 through 123.

20. Use according to claim 16 wherein said adenovirus is d1922/947.

21. Use according to claim 16 wherein said adenovirus is d1107.

22. A pharmaceutical composition comprising a Rb binding site adenoviral mutant, wherein said mutation is in the E1A-CR2 region and comprises a deletion or substitution of one or more of (a) amino acids 122 through 129, or (b) amino acids 111 through 123, in a physiological solution.

23. A pharmaceutical composition as described in claim 22 wherein said adenoviral mutant is d1922/947.

24. A pharmaceutical composition as described in claim 22 wherein said adenoviral mutant is d1107.

25. A pharmaceutical composition as described in claim 22 wherein said adenoviral mutant is pm928.

## Patentansprüche

1. In-vitro-Verfahren zum selektiven Töten eines Großteils von sich teilenden Zellen in einer Zellpopulation, die sich teilende und ruhende Zellen umfasst, worin dieses Verfahren das Kontaktieren der Zellpopulation unter infektiösen Bedingungen mit einem replikationsfähigen Adenovirus, das eine Mutation in der E1A-RB-Familienmitgliedbindungsregion dieses Adenovirus umfasst, und das ausreichend lange Warten umfasst, sodass dieses Adenovirus die Zellpopulation infizieren kann, worin sich die Mutation in der E1A-CR2-Region befindet und eine Deletion oder Substitution einer oder mehrerer (a) der Aminosäuren 122 bis 129; oder (b) der Aminosäuren 111 bis 123 umfasst.

2. Verfahren nach Anspruch 1, worin die sich teilenden Zellen Krebszellen sind.

3. Verfahren nach Anspruch 2, worin die sich teilenden Zellen Endothelzellen sind.

4. Verfahren nach Anspruch 3, worin die ruhenden Zellen Endothelzellen sind.

5. Verfahren nach einem der Ansprüche 1 bis 4, worin das Adenovirus dl922/947 ist.

6. Verfahren nach einem der Ansprüche 1 bis 4, worin das Adenovirus dl1107 ist.

7. Verfahren nach einem der Ansprüche 1 bis 4, worin das Adenovirus pm928 ist.

8. In-vitro-Verfahren zum Töten von sich teilenden Endothelzellen mit wesentlich geringerem Töten von ruhenden Endothelzellen in einer Zellpopulation, die sich teilende und ruhende Endothelzellen umfasst, worin das Verfahren das Kontaktieren der Zellpopulation unter infektiösen Bedingungen mit einem replikationsfähigen Adenovirus, worin dieses Adenovirus eine Mutation in einer E1A-CR2-RB-Familienmitgliedbindungsregion von diesem Adenovirus umfasst, und das ausreichend lange Warten umfasst, sodass das mutierte Adenovirus die Zellpopulation infizieren kann, worin das mutierte Adenovirus in den sich teilenden Zellen zu höheren Titern als Wildtyp-Adenovirus repliziert.

9. In-vitro-Verfahren zum selektiven Töten eines Großteils von sich teilenden Endothelzellen und Krebszellen im Vergleich zu ruhenden Endothelzellen in einer Zellpopulation, die diese drei Zelltypen umfasst, worin das Verfahren das Kontaktieren dieser Zellpopulation unter infektiösen Bedingungen mit einem replikationsfähigen Adenovirus, das eine Mutation in einer E1A-CR2-RB-Familienmitgliedbindungsregion von diesem Adenovirus umfasst, und das ausreichend lange Warten umfasst, sodass das mutierte Adenovirus die Zellpopulation infizieren kann.

10. Verfahren nach Anspruch 9, worin die sich teilenden Endothelzellen Mikrogefäßendothelzellen sind.

11. Verfahren nach Anspruch 10, worin die Adenovirusmutation eine Mutation in der E1A-CR2-Region umfasst.

12. Verwendung eines replikationsfähigen Adenovirus, das eine Mutation in der E1A-CR2-Region von diesem Adenovirus umfasst, worin die Mutation eine Deletion oder Substitution einer oder mehrerer (a) der Aminosäuren 122 bis 129; oder (b) der Aminosäuren 111 bis 123 umfasst; zur Herstellung eines Medikamentes zur Verwendung in der Behandlung oder Prävention einer Erkrankung unkontrollierten oder hyperproliferativen Zellwachstums.

13. Verwendung nach Anspruch 12, worin das Adenovirus dl922/947 ist.

14. Verwendung nach Anspruch 12, worin das Adenovirus dl1107 ist.

15. Verwendung nach Anspruch 12, worin das Adenovirus pm928 ist.

16. Verwendung eines replikationsfähigen Adenovirus, das eine Mutation in der E1A-RB-Familienmitgliedbindungsregion umfasst, zur Herstellung eines Medikamentes zur Kontrolle von Angiogenese in einem Tier durch Töten von sich teilenden Mikrogefäßendothelzellen.

17. Verwendung nach Anspruch 16, worin die E1A-RB-Familienmitgliedbindungsregion dieses Adenovirus in der E1A-CR2-Region liegt.

18. Verwendung nach Anspruch 17, worin die Mutation in der E1A-CR2-Region eine Deletion oder Substitution einer oder mehrerer der Aminosäuren 122 bis 129 umfasst.

19. Verfahren nach Anspruch 18, worin die Mutation in der E1A-CR2-Region eine Deletion oder Substitution einer oder mehrerer Aminosäuren 111 bis 123 umfasst.

20. Verfahren nach Anspruch 16, worin das Adenovirus dl922/947 ist.

21. Verfahren nach Anspruch 16, worin das Adenovirus d1107 ist.

22. Pharmazeutische Zusammensetzung, umfassend eine Rb-Bindungsstellen-Adenovirusmutante, worin die Mutation in der E1A-CR2-Region liegt und eine Deletion oder Substitution einer oder mehrerer (a) der Aminosäuren 122 bis 129, oder (b) der Aminosäuren 111 bis 123 umfasst, in einer physiologischen Lösung.

23. Pharmazeutische Zusammensetzung nach Anspruch 22, worin die Adenovirusmutante dl922/947 ist.

24. Pharmazeutische Zusammensetzung nach Anspruch 22, worin die Adenovirusmutante d1107 ist.

25. Pharmazeutische Zusammensetzung nach Anspruch 22, worin die Adenovirusmutante pm928 ist.

## Revendications

1. Méthode in vitro pour sensiblement et sélectivement tuer des cellules se divisant dans une population de cellules comprenant des cellules se divisant et au repos, ladite méthode comprenant la mise en contact de ladite population de cellules en conditions infectieuses avec un adénovirus compétent en réplication comprenant une mutation dans la région liant un membre de la famille E1A RB dudit adénovirus, et en laissant suffisamment de temps pour que ledit adénovirus infecte ladite population de cellules, où ladite mutation est dans la région E1A-CR2 et comprend une délétion ou substitution d'un ou plusieurs de (a) acides aminés 122 à 129 ; ou (b) acides aminés 111 à 123.

2. Méthode telle que décrite à la revendication 1, où lesdites cellules se divisant sont des cellules de cancer.

3. Méthode telle que décrite à la revendication 2, où les cellules se divisant sont des cellules endothéliales.

4. Méthode telle que décrite à la revendication 2, où les cellules au repos sont des cellules endothéliales.

5. Méthode telle que décrite dans l'une quelconque des revendications 1 à 4, où ledit adénovirus est d1922/947.

6. Méthode telle que décrite dans l'une quelconque des revendications 1 à 4, où ledit adénovirus est d11107.

7. Méthode telle que décrite dans l'une quelconque des revendications 1 à 4, où ledit adénovirus est pm928.

8. Méthode in vitro pour tuer des cellules endothéliales se divisant avec sensiblement moins de mort des cellules endothéliales au repos, dans une population de cellules comprenant des cellules endothéliales se divisant et au repos, ladite méthode comprenant la mise en contact de ladite population de cellules en conditions infectieuses avec un adénovirus, et compétent en réplication, ledit adénovirus comprenant une mutation dans une région de liaison d'un membre de la famille E1A CR2 RB dudit adénovirus et en laissant suffisamment de temps pour que ledit adénovirus mutant infecte ladite population de cellules, où ledit adénovirus mutant se réplique à des titres plus élevés dans lesdites cellules se divisant que l'adénovirus du type sauvage.

9. Méthode in vitro pour tuer sensiblement et sélectivement des cellules endothéliales se divisant et des cellules de cancer, en comparaison avec les cellules endothéliales au repos dans une population de cellules comprenant lesdits trois types de cellules, ladite méthode comprenant la mise en contact de ladite population de cellules en conditions infectieuses avec un adénovirus compétent en réplication comprenant une mutation dans une région de liaison d'un membre de la famille E1A CR2 RB dudit adénovirus, et en laissant suffisamment de temps pour que ledit adénovirus mutant infecte ladite population de cellules.

10. Méthode telle que décrite à la revendication 9, où lesdites cellules endothéliales se divisant sont des cellules endothéliales microvasculaires.

11. Méthode telle que décrite à la revendication 10, où ladite mutation de l'adénovirus comprend une mutation dans la région E1A-CR2.

12. Utilisation d'un adénovirus compétent en réplication comprenant une mutation de la région E1A CR2 dudit adénovirus, ladite mutation comprenant une délétion de substitution d'un ou plusieurs de (a) acides aminés 122 à 129 ; ou (b) acides aminés 111 à 123 ; dans la fabrication d'un médicament utilisé dans le traitement ou la prévention d'une maladie d'une croissance non contrôlée ou hyperproliférative des cellules.

13. Utilisation selon la revendication 12, où ledit adénovirus est d1922/947.

14. Utilisation selon la revendication 12, où ledit adénovirus est d11107.

15. Utilisation selon la revendication 12, où ledit adénovirus est pm928.

16. Utilisation d'un adénovirus compétent en réplication comprenant une mutation dans la région de liaison d'un membre de la famille E1A RB, dans la fabrication d'un médicament pour contrôler l'angiogénèse chez un animal, en tuant les cellules endothéliales microvasculaires se divisant.

17. Utilisation selon la revendication 16, où ladite région liant un membre de la famille E1A RB dudit adénovirus est dans la région E1A CR2.

18. Utilisation selon la revendication 17, où ladite mutation dans la région E1A CR2 comprend une délétion ou substitution d'un ou plusieurs acides aminés 122 à 129.

19. Utilisation selon la revendication 18, où ladite mutation dans la région E1A-CR2 comprend une délétion ou substitution d'un ou plusieurs acides aminés 111 à 123.

20. Utilisation selon la revendication 16, où ledit adénovirus est d1922/947.

21. Utilisation selon la revendication 16, où ledit adénovirus est d1107.

22. Composition pharmaceutique comprenant un mutant adénoviral au site de liaison de Rb, où ladite mutation est dans la région E1A-CR2 et comprend une délétion ou substitution d'un ou plusieurs de (a) acides aminés 122 à 129 ou (b) acides aminés 111 à 123, dans une solution physiologique.

23. Composition pharmaceutique telle que décrite à la revendication 22, où ledit mutant adénoviral est d1922/947.

24. Composition pharmaceutique telle que décrite à la revendication 22, où ledit mutant adénoviral est d1107.

25. Composition pharmaceutique telle que décrite à la revendication 22, où ledit mutant adénoviral est pm928.
